# EUROPÄISCHE PATENTSCHRIFT

(11) **EP 3 536 349 B1**
(45) Veröffentlichungstag und Bekanntmachung des Hinweises auf die Patenterteilung: **16.11.2022**
(21) Anmeldenummer: 19158232.9
(22) Anmeldetag: 20.02.2019
(51) Int. Cl.: G01N 21/77, A61L 2/26

(54) **ANALYSEEINHEIT**
ANALYSIS UNIT
UNITÉ D'ANALYSE

(30) Priorität: 07.03.2018 DE 102018105174
(43) Veröffentlichungstag der Anmeldung: 11.09.2019
(73) Patentinhaber: PreSens Precision Sensing GmbH, 93053 Regensburg (DE)
(72) Erfinder: RIECHERS, Daniel, 93051 Regensburg (DE)
(74) Vertreter: Reichert & Lindner Partnerschaft Patentanwälte

(56) Entgegenhaltungen:
- WO-A1-2010/145747
- DE-A1-102015 116 355
- DE-A1-102016 124 647
- DE-B3-102016 000 997

## Beschreibung

Die Erfindung betrifft eine Analyseeinheit für die chemische Sensorik, insbesondere eine Analyseeinheit mit mindestens einem eingelagerten Sensor.

Der Einsatz von Sensoren zum Nachweis eines Analyten in einer Probe ist bekannt, siehe beispielsweise die deutschen Patentanmeldungen DE 10 2010 061 182 A1, DE 10 2011 055 272 A1, DE 10 2014 107 837 A1 oder das deutsche Patent 10 2013 108 659 B3, sowie die darin zitierten Dokumente aus dem Stand der Technik.

Derartige Sensoren beinhalten eine Sensorsubstanz, welche auf einen Analyten der Probe, also einen in der Probe nachzuweisenden Stoff, sensitiv ist. Beispielweise kann die Sensorsubstanz ein optisches Verhalten aufweisen, welches in direkter oder indirekter Weise durch den Analyten beeinflusst wird. Aus der Auswertung des optischen Verhaltens kann quantitativ auf den Analyten geschlossen werden. Derartige Sensoren, ihr Aufbau, für jeweilige Analyten und Einsatzbedingungen geeignete Sensorsubstanzen und Messverfahren zur Auswertung des optischen Verhaltens des Sensors bzw. der Sensorsubstanz sind dem Fachmann bekannt, zum Beispiel aus dem oben genannten Stand der Technik.

Bei etlichen der Sensoren ist es vor ihrem Einsatz zu einer Messung erforderlich, den Sensor quellen zu lassen, d.h. der Sensor muss mit einer geeigneten Lösung durchfeuchtet werden, um die Einsatzbereitschaft herzustellen. Diese Vorbereitungsphase vor der Messung ist z.B. nachteilig, wenn kurzfristig Messergebnisse erforderlich sind, und allgemein für einen Benutzer umständlich.

Die europäische Patentanmeldung EP 0 354 895 A2 betrifft ein Einweg-Messelement, in dem vor einer Messung ein Sensor in einem Messbereich eines beidseitig verschlossenen Messkanals angeordnet ist. Der Messkanal ist mit einem Kalibrier- und Lagermedium gefüllt. Zur Messung wird das Kalibier- und Lagermedium durch eine in den Messkanal strömende Probe verdrängt. Der Sensor kann ein optischer Sensor sein. Der Verschluss der Messkanals kann durch eine durchstechbare Membran verwirklicht werden. Bei ähnlichem Aufbau wird in der europäischen Patentanmeldung EP 0 460 343 A2 vorgeschlagen, das Lagermedium zunächst durch ein Trennmedium, bei dem es sich um ein Kalibriermedium handeln kann, zu verdrängen, und anschließend das Trennmedium durch die Probe zu verdrängen.

Das deutsche Patent DE 10 2016 000 997 B3 offenbart ein System zum Aufbewahren und/oder Kalibrieren eines Sensors. Dabei bildet eine flexible Hülle einen Raum, in dem der Sensor angeordnet ist. Fluide können in den Raum zugeführt und daraus abgeführt werden. Bei den Fluiden kann es sich um Fluide zum Aufbewahren oder Kalibrieren des Sensors handeln. Um den Sensor mit einer Probe in Kontakt zu bringen, wird eine Menge eines Fluids in den Raum eingeführt, die ausreichend ist, dass die flexible Hülle sich öffnet, beispielsweise platzt.

Die deutsche Offenlegungsschrift DE 10 2015 116 355 A1 beschreibt eine Sensoranordnung, bei der ein Sensor im Innenraum eines Gehäuses angeordnet ist. Das Gehäuse ist an einem Behälter für die Probe angebracht. Der Innenraum des Gehäuses ist durch eine Gehäusewandung von dem Innenraum des Behälters getrennt. Um den Sensor mit der Probe in Kontakt zu bringen, wird die Gehäusewandung zerstört, durch Erzeugen eines Überdrucks oder Unterdrucks in dem Gehäuseinnenraum oder durch Zusammenwirkung etwa mit einem geeignet geformten Vorsprung eines Adapters für das Gehäuse in der Behälterwand.

Das deutsche Gebrauchsmuster DE 20 2010 006 207 U1 betrifft verschiedene Anordnungen, mit denen es möglich ist, in einer Messanordnung einen einen Sensor enthaltenden Bereich zumindest zeitweilig von einem Probenbereich zu trennen. Zweck ist, den Sensor während einer Sterilisierung zu schützen. In bestimmten Ausführungen ist der Sensor dabei von einem wässrigen Reduktionsmittel umgeben. Eine derartige Anordnung ist auch in der WO 2010/145747 A1 beschrieben.

Die US-Patentanmeldung US 2012/0267518 A1 offenbart einen Sensor, der zum Schutz bei einer Sterilisierung in einem Lagerbereich angeordnet ist und aus dem Lagerbereich in einen Probenbereich verschoben werden kann.

Die deutsche Offenlegungsschrift DE 10 2016 124 647 A1 betrifft ebenfalls den Schutz eines Sensors bei der Sterilisierung. Der Sensor ist in einem abgegrenzten Bereich geringer Luftfeuchtigkeit angeordnet. Um den Sensor nach der Sterilisierung mit der Probe in Kontakt zu bringen, kann eine Wandung des abgegrenzten Bereichs mit einem Sensorträger, auf welchem der Sensor angeordnet ist, durchstoßen werden.

Der Schutz bei der Sterilisierung ist ein wichtiger Aspekt bei der Anwendung von Sensoren, vermag aber nicht, das Problem der Wartezeit bis zur Einsatzfähigkeit zu lösen. Die im Stand der Technik hierzu vorgeschlagene Lösung der Lagerung des von einem Lagermedium umgebenen Sensors im Messbereich eines Messkanals erfordert die Anbindung von Behältern oder Leitungen für die Probe an den Messkanal. Dies gestaltet sich in der Handhabung aufwändig.

Aufgabe der Erfindung ist es daher, eine Analyseeinheit mit mindestens einem Sensor bereitzustellen, bei der eine Wartezeit bis zur Einsatzbereitschaft des Sensors bzw. der Analyseeinheit nicht erforderlich ist, wobei außerdem die Analyseeinheit in der Handhabung gegenüber dem Stand der Technik vereinfacht sein soll.

Diese Aufgabe wird gelöst durch eine Analyseeinheit gemäß Anspruch 1. Die Unteransprüche enthalten vorteilhafte Ausgestaltungen.

Die erfindungsgemäße Analyseeinheit umfasst mindestens einen Sensor. Die Analyseeinheit umfasst einen Lagerbereich und einen von diesem durch eine Barriere getrennten Probenbereich. Im Lagerbereich ist ein Lagermedium für den Sensor enthalten. Der Probenbereich ist zur Aufnahme einer zu untersuchenden Probe vorgesehen. In einem Lagerzustand der Analyseeinheit, also einem Zustand, in dem die Analyseeinheit aufbewahrt werden kann, bis sie für eine Probenanalyse benötigt wird, ist der mindestens eine Sensor in dem Lagerbereich innerhalb des Lagermediums angeordnet. Im Lagermedium kann der mindestens eine Sensor quellen, d.h. von dem Lagermedium durchfeuchtet werden. Dies bedeutet, dass bei der erfindungsgemäßen Analyseeinheit der mindestens eine Sensor im durchfeuchteten, einsatzbereiten Zustand gelagert werden kann. Die Wartezeit bis zum Abschluss des Durchfeuchtens vor einer Probenanalyse entfällt daher. Als Lagermedium kommen damit insbesondere solche Medien, z.B. wässrige Lösungen, in Betracht, die nach dem Stand der Technik verwendet werden, um einen jeweiligen Sensor vor einer Messung zu durchfeuchten. Für eine Probenanalyse muss die Analyseeinheit in einen Messzustand versetzt werden, d.h. in einen Zustand, in dem der mindestens eine Sensor mit einer Probe in Kontakt bringbar ist. Die erfindungsgemäße Analyseeinheit ist dazu derart ausgebildet, dass der mindestens eine Sensor aus dem Lagerbereich der Analyseeinheit in den Probenbereich der Analyseeinheit verschoben werden kann, wobei die Barriere zwischen dem Lagerbereich und dem Probenbereich durchbrochen wird. Dabei kann der Probenbereich bereits eine Probe enthalten oder erst später mit einer Probe gefüllt werden.

In einer Ausführungsform ist der mindestens eine Sensor auf einem Sensorträger angeordnet. Der Sensorträger ist hierbei dazu ausgebildet, die Barriere zwischen dem Lagerbereich und dem Probenbereich zu durchbrechen. Zu diesem Zweck kann der Sensorträger beispielsweise einen oder mehrere geeignet geformte Vorsprünge aufweisen, die die Barriere durchstechen oder durchschneiden können. Die Vorsprünge können hierzu etwa Spitzen oder Schneidkanten aufweisen.

In einer Ausführungsform ist der mindestens eine Sensor ein optischer Sensor, d.h. der Sensor ist dazu ausgebildet, optisch angeregt zu werden und eine von einem Analyten abhängige optische Antwort zu liefern, welche nach Erfassung und Auswertung einen quantitativen Rückschluss auf den Analyten erlaubt. Beispielsweise können Konzentration oder Partialdruck des Analyten in der Probe bestimmt werden. Der Sensorträger ist dabei transparent für das bei der optischen Anregung und der optischen Antwort involvierte Licht. Dies bedeutet, der Sensorträger lässt zumindest so viel des für die optische Anregung verwendeten Lichts und so viel des Lichts der optischen Antwort des Sensors durch, dass die quantitative Bestimmung des Analyten in für den jeweiligen konkreten Einsatzzweck ausreichender Weise möglich ist. Beispielsweise kann der Sensorträger wenigstens 50% der auf ihn einfallenden Lichtintensität durchlassen, bevorzugt mindestens 90%. Nichteinschränkende Beispiele für Analyten sind etwa O₂, CO₂ oder pH-Wert. Allgemein lässt sich die erfindungsgemäße Analyseeinheit zum quantitativen Nachweis aller Analyten einsetzen, für die geeignete Sensoren existieren.

In einer vorteilhaften Weiterbildung ist im Sensorträger ein Kanal für einen Lichtleiter vorgesehen. Dadurch kann der Sensorträger an einen Lichtleiter gekoppelt werden und Licht zur Anregung des Sensors sowie Licht der optischen Antwort des Sensors können durch den Lichtleiter laufen.

In einer Ausführungsform der Analyseeinheit ist der Lagerbereich mit dem Probenbereich durch ein Zwischenelement verbunden. In dem Zwischenelement ist eine Aufnahme für den Sensorträger vorgesehen, um den Sensorträger im Messzustand aufzunehmen. Das Zwischenelement ist vorzugsweise so angeordnet, dass die Barriere zwischen dem Lagerbereich und dem Probenbereich das Zwischenelement von dem Lagerbereich trennt. Wird dann der Sensorträger verschoben, um die Analyseeinheit in den Messzustand zu versetzen, so dass der Sensorträger die Barriere durchbricht, so wird der Sensorträger in der im Zwischenelement ausgebildeten Aufnahme aufgenommen. Der Sensor befindet sich dann in einer Position, in welcher er mit einer Probe in Kontakt gebracht werden kann.

Wird die Barriere durch den Sensorträger durchbrochen, so verbleiben Bruchstücke der Barriere, entweder lose oder noch mit der Analyseeinheit verbunden, in der Umgebung des Sensorträgers. Dies kann eine Abdichtung zwischen dem Sensorträger und der Aufnahme im Zwischenelement beeinträchtigen. In einer vorteilhaften Ausführungsform ist daher in der Aufnahme für den Sensorträger wenigstens ein Freiraum zur Aufnahme wenigstens eines Teils der durchbrochenen Barriere vorgesehen, so dass Bruchstücke der Barriere nicht mehr unkontrolliert zwischen Sensorträger und Zwischenelement eingeklemmt werden.

In einer Ausführungsform ist in dem Zwischenelement ein Dichtring, etwa ein O-Ring, vorgesehen. Der Dichtring dient dazu, im Messzustand einen Spalt zwischen Zwischenelement und Sensorträger abzudichten. Dadurch kann insbesondere eine Probe im Probenbereich vor dem Lagermedium geschützt werden.

Um bei einer Analyseeinheit im Messzustand eine Verschiebung des Sensorträgers zurück in den Lagerbereich zu verhindern, kann der Sensorträger geeignet in dem Zwischenelement fixiert werden. In einer Ausführungsform sind dazu der Sensorträger und das Zwischenelement ausgebildet, eine Rastverbindung miteinander einzugehen. Bei einer, durch die Rastverbindung oder allgemeiner durch eine geeignete Fixierung verhinderten, Verschiebung des Sensorträgers zurück in den Lagerbereich würde, da die Barriere durchbrochen ist, die Probe mit dem Lagermedium kontaminiert werden. Außerdem befände sich der mindestens eine Sensor nicht mehr in der für die Messung vorgesehenen Position.

In einer Ausführungsform ist der Sensorträger im Lagerbereich gegen ein unbeabsichtigtes Verschieben in den Probenbereich gesichert. Dazu kann etwa eine Feder vorgesehen sein, welche auf den Sensorträger eine Kraft ausübt, welche von der Barriere weg gerichtet ist. Um die Barriere zu durchbrechen, müsste beim Verschieben des Sensorträgers zumindest die Federkraft überwunden werden. In anderen Ausführungsformen kann der Sensorträger beispielsweise durch einen Sicherungsstift oder eine Lasche an einem Gehäuseteil der Analyseeinheit gegen unbeabsichtigtes Verschieben fixiert werden. Sicherungsstift oder Lasche müssten entfernt oder zerstört werden, ehe die Barriere durchbrochen werden kann.

Die Barriere zwischen dem Lagerbereich und dem Probenbereich ist in einer Ausführungsform durch einen Teil einer Wandung des Lagerbereichs gebildet. In dieser Ausführungsform aber auch in anderen Ausführungsformen kann die Barriere zwischen dem Lagerbereich und dem Probenbereich eine Sollbruchstelle aufweisen.

In einer Ausführungsform umfasst der Lagerbereich einen Ausweichbereich. Der Ausweichbereich ist dazu vorgesehen, Lagermedium aufzunehmen, welches bei Verschiebung des Sensors aus dem Lagerbereich in den Probenbereich verdrängt wird.

In einer Ausführungsform ist der Lagerbereich durch eine Folie begrenzt. In diesem Fall kann die Barriere zwischen dem Lagerbereich und dem Probenbereich durch ein Stück der Folie gegeben sein. Eine Sollbruchstelle in der Barriere kann erzeugt werden, indem das betreffende Stück Folie durch einen Laser geschwächt wird. Bei der Folie kann es sich insbesondere um eine Verbundfolie handeln. Ein Ausweichbereich kann etwa dadurch erzeugt werden, dass nur ein Teil des von der Folie umschlossenen Volumens mit Lagermedium gefüllt wird, während ein oder mehrere andere Bereiche des Volumens bei der Befüllung evakuiert gehalten werden, etwa indem diese Bereiche während des Befüllens eingeklemmt werden.

In einer Ausführungsform ist im Lagermedium eine definierte Osmolalität eingestellt. Dies ist von Vorteil, da etliche Sensoren zur Herstellung von Einsatzbereitschaft nicht nur durchfeuchtet, sondern auch auf eine bestimmte Osmolalität eingestellt werden müssen. In vielen Fällen handelt es sich dabei um die Osmolalität einer Probe, die mit der Analyseeinheit untersucht werden soll. Ist eine erfindungsgemäße Analyseeinheit etwa zur Untersuchung von Blutproben vorgesehen, so kann die Osmolalität des Lagermediums auf die Osmolalität von Blut eingestellt sein. Beispielsweise kann dann als Lagermedium isotonische Kochsalzlösung verwendet werden.

In einer Ausführungsform dient das Lagermedium zusätzlich als Kalibriermedium. Beispielsweise kann im Lagermedium ein definierter Wert eines nachzuweisenden Analyten eingestellt sein. Bei einer derartigen Ausgestaltung der erfindungsgemäßen Analyseeinheit kann, während sich der Sensor noch im Lagermedium befindet, eine Ein-Punkt-Kalibrierung des Sensors vorgenommen werden.

In einer Ausführungsform der erfindungsgemäßen Analyseeinheit bildet der Probenbereich ein Durchflusselement. Ebenso kann der Probenbereich aber durch einen Behälter, etwa eine Flasche, eine Röhre, einen Beutel gegeben sein, in welchen die Probe zu Beginn der Messung eingefüllt wird und während der Messung darin verbleibt. Es ist auch denkbar, dass in dem Behälter, welcher den Probenbereich bildet, ein Mittel zur Umwälzung der Probe, etwa zum Rühren der Probe, vorgesehen ist.

Im Folgenden werden die Erfindung und ihre Vorteile an Hand der beigefügten schematischen Zeichnungen, welche Ausführungsbeispiele der Erfindung zeigen, näher erläutert.
- Figur 1: zeigt eine erfindungsgemäße Analyseeinheit im Lagerzustand.
- Figur 2: zeigt die erfindungsgemäße Analyseeinheit aus Figur 1 im Messzustand.
- Figur 3: zeigt eine weitere Ausführungsform einer erfindungsgemäßen Analyseeinheit im Lagerzustand.

Die Zeichnungen sind dabei nicht als Beschränkung der Erfindung auf die dargestellten Ausführungsbeispiele anzusehen.

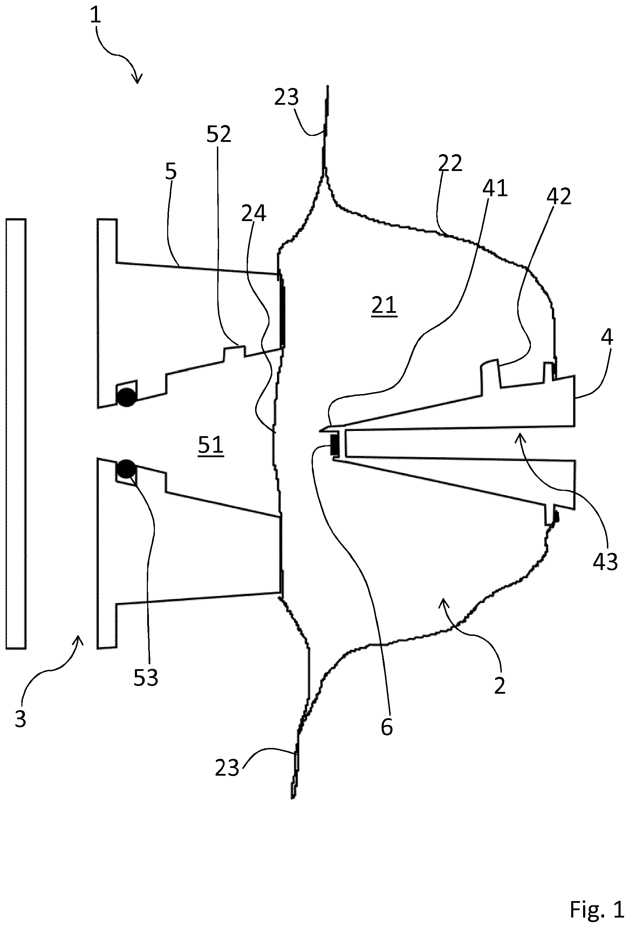

**Fig. 1** zeigt eine erfindungsgemäße Analyseeinheit 1 im Lagerzustand. Die Analyseeinheit 1 umfasst einen Lagerbereich 2, in dem ein Lagermedium 21 enthalten ist, und einen Probenbereich 3, welcher in der gezeigten Ausführungsform ein Durchflusselement bildet. Ein Sensor 6 ist in dem Lagermedium 21 auf einem Sensorträger 4 angeordnet. Der Lagerbereich 2 ist mit dem Probenbereich 3 durch ein Zwischenelement 5 verbunden.

Der Lagerbereich 2 ist durch eine Verbundfolie 22 begrenzt. Die Verbundfolie 22 umschließt ebenfalls Ausweichbereiche 23, in welche das Lagermedium bei Verschiebung des Sensorträgers 4 verdrängt werden kann; auf diese Weise wird ein unkontrolliertes Bersten der Verbundfolie 22 vermieden. Ein Teilbereich der Verbundfolie 22 bildet eine Barriere 24 zwischen dem Lagerbereich 2 und dem Probenbereich 3. Die Barriere 24 kann durch den Sensorträger 4 durchstoßen werden. Zu diesem Zweck ist am Sensorträger 4 eine Spitze 41 ausgebildet. Im Zwischenelement 5 ist eine Aufnahme 51 vorgesehen, in welche der Sensorträger 4 im Messzustand aufgenommen werden kann. Am Sensorträger 4 ist eine Rastnase 42 ausgebildet, welche dazu vorgesehen ist, mit einer Rastkerbe 52 im Zwischenelement 5 zusammenzuwirken, um den Sensorträger 4 in der Aufnahme 51 durch eine Rastverbindung zu fixieren. Ebenso wäre es denkbar, dass am Sensorträger 4 eine Rastkerbe und in der Aufnahme 51 eine Rastnase ausgebildet ist, um den Sensorträger 4 in der Aufnahme 51 durch eine Rastverbindung zu fixieren. Ferner ist ein Dichtring 53 vorgesehen, um den Bereich aus Aufnahme 51 und Lagerbereich 2 im Messzustand gegen den Probenbereich 3 abzudichten. Die Verbundfolie 22 ist mit dem Sensorträger 4 und dem Zwischenelement 5 verschweißt oder verklebt. Im Sensorträger 4 ist ein Kanal 43 für einen Lichtleiter ausgebildet.

In der gezeigten Ausführungsform sind das Zwischenelement 5 und der als Durchflusselement ausgebildete Probenbereich 3 einstückig aus einem Kunststoff gebildet; beispielsweise aus HDPE (High-Density-Polyethylen), PC (Polycarbonat), PS (Polystyrol) oder PSU (Polysulfon). Der Sensorträger 4 kann ebenfalls aus solch einem Kunststoff hergestellt sein. Die Verbundfolie 22 kann beispielsweise drei Schichten umfassen, etwa PET (Polyethylenterephthalat), Al (Aluminium) und PE (Polyethylen). Dabei dient Aluminium als Gasbarriere und hält beispielsweise bei wässrigem Lagermedium die Verdunstung aus dem Lagerbereich unter 0,5% H₂O in zwei Jahren. PET sorgt für Stabilität und PE sorgt für gute Verschweißbarkeit der Folie.

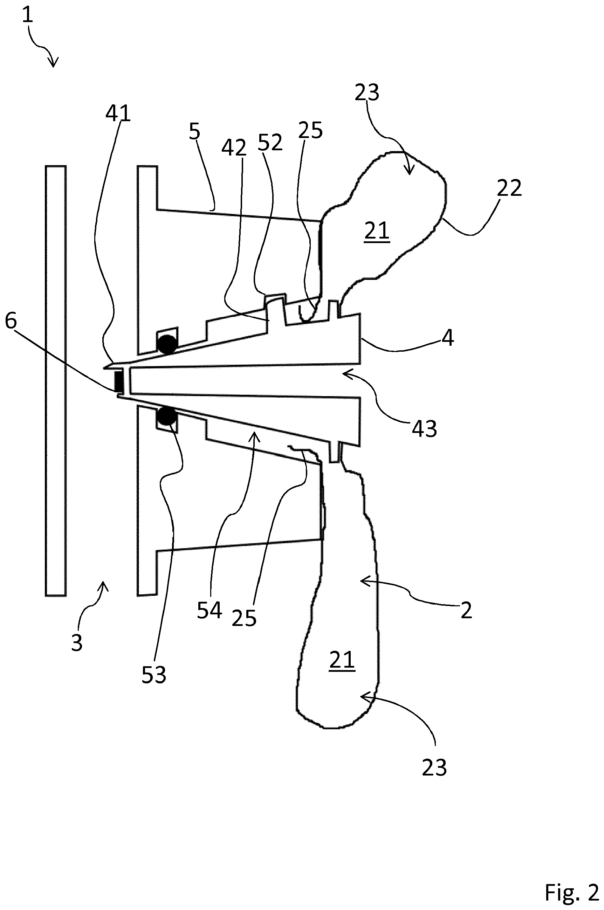

**Fig. 2** zeigt die erfindungsgemäße Analyseeinheit 1 aus Fig. 1 im Messzustand. Die dargestellten Elemente wurden bereits zu Fig. 1 erläutert. Der Sensorträger 4 mit dem Sensor 6 wurde in Richtung des Probenbereichs 3 verschoben, hat dabei unterstützt durch die Spitze 41 die Barriere 24 (siehe Fig. 1) durchstoßen und sitzt nun in der Aufnahme 51 (siehe Fig. 1) des Zwischenelements 5; durch eine Rastverbindung zwischen Rastnase 42 und Rastkerbe 52 wird der Sensorträger 4 in dieser Position gehalten. Bruchstücke 25 der Barriere 24 finden in einem Freiraum 54 zwischen Sensorträger 4 und Zwischenelement 5 Platz; auf diese Weise wird ein unkontrolliertes Einklemmen der Bruchstücke 25 zwischen Sensorträger 4 und Zwischenelement 5 vermieden. Der Sensor 6 befindet sich im Probenbereich 3 und kann dort mit einer Probe in Kontakt gebracht werden. Beim Verschieben des Sensorträgers 4 wurde Lagermedium 21 in die Ausweichbereiche 23 verdrängt. Dichtring 53 dichtet den Zwischenraum zwischen Sensorträger 4 und Zwischenelement 5 ab.

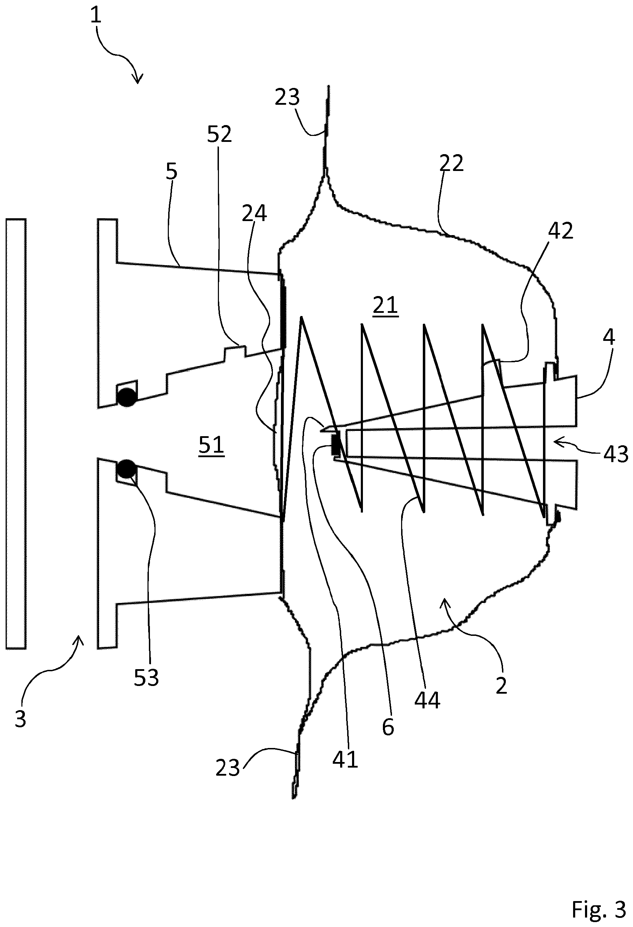

**Fig. 3** zeigt eine weitere Ausführungsform einer erfindungsgemäßen Analyseeinheit 1 im Lagerzustand. Die dargestellte Analyseeinheit 1 entspricht weitgehend der in Fig. 1 gezeigten; in Fig. 1 wurden auch bereits die meisten der dargestellten Elemente diskutiert. Im Unterschied zu der in Fig. 1 gezeigten Ausführungsform ist in dieser Ausführungsform eine Feder 44 vorgesehen, welche sich sowohl am Sensorträger 4 als auch am Zwischenelement 5 abstützt. Dadurch übt die Feder 44 eine Kraft auf den Sensorträger 4 weg von der Barriere 24 aus. Um die Barriere 24 durch Verschieben des Sensorträgers 4 zu durchbrechen, muss zunächst die Federkraft der Feder 44 überwunden werden. Auf diese Weise ist die Analyseeinheit 1 gegen ein unbeabsichtigtes Durchbrechen der Barriere 24 mit dem Sensorträger 4 geschützt. Die Feder 44 kann insbesondere als Schraubenfeder um den Sensorträger 4 herumlaufen.

### Bezugszeichenliste

- 1: Analyseeinheit
- 2: Lagerbereich
- 3: Probenbereich
- 4: Sensorträger
- 5: Zwischenelement
- 6: Sensor
- 21: Lagermedium
- 22: Verbundfolie
- 23: Ausweichbereich
- 24: Barriere
- 25: Bruchstück
- 41: Spitze
- 42: Rastnase
- 43: Kanal für Lichtleiter
- 44: Feder
- 51: Aufnahme
- 52: Rastkerbe
- 53: Dichtring
- 54: Freiraum

## Patentansprüche

1. Analyseeinheit (1) mit mindestens einem Sensor (6),
wobei in einem Lagerzustand der Analyseeinheit (1) der mindestens eine Sensor (6) in einem Lagermedium (21) angeordnet und von dem Lagermedium (21) durchfeuchtet ist;
wobei in einem Messzustand der Analyseeinheit (1) der mindestens eine Sensor (6) mit einer Probe in Kontakt bringbar ist;
**dadurch gekennzeichnet, dass**
die Analyseeinheit (1) aus dem Lagerzustand in den Messzustand versetzbar ist, indem der mindestens eine Sensor (6) aus einem das Lagermedium (21) enthaltenden Lagerbereich (2) der Analyseeinheit (1) in einen Probenbereich (3) der Analyseeinheit (1) verschoben wird, wobei eine Barriere (24) zwischen dem Lagerbereich (2) und dem Probenbereich (3) durchbrochen wird.

2. Analyseeinheit (1) nach Anspruch 1, wobei der mindestens eine Sensor (6) auf einem Sensorträger (4) angeordnet ist, welcher dazu ausgebildet ist, die Barriere (24) zwischen dem Lagerbereich (2) und dem Probenbereich (3) zu durchbrechen.

3. Analyseeinheit (1) nach Anspruch 2, wobei der mindestens eine Sensor (6) dazu ausgebildet ist, optisch angeregt zu werden und eine von einem Analyten abhängige optische Antwort zu liefern, und der Sensorträger (4) transparent für das bei der optischen Anregung und der optischen Antwort involvierte Licht ist.

4. Analyseeinheit (1) nach Anspruch 3, wobei im Sensorträger (4) ein Kanal (43) für einen Lichtleiter vorgesehen ist.

5. Analyseeinheit (1) nach einem der Ansprüche 2 bis 4, wobei der Lagerbereich (2) mit dem Probenbereich (3) durch ein Zwischenelement (5) verbunden ist, in dem eine Aufnahme (51) für den Sensorträger (4) vorgesehen ist, um den Sensorträger (4) im Messzustand aufzunehmen.

6. Analyseeinheit (1) nach Anspruch 5, wobei in der Aufnahme (51) für den Sensorträger (4) wenigstens ein Freiraum (54) zur Aufnahme wenigstens eines Teils der durchbrochenen Barriere (24) vorgesehen ist.

7. Analyseeinheit (1) nach Anspruch 5 oder 6, wobei in dem Zwischenelement (5) ein Dichtring (53) vorgesehen ist, um das Zwischenelement (5) gegen den Sensorträger (4) abzudichten.

8. Analyseeinheit (1) nach einem der Ansprüche 5 bis 7, wobei der Sensorträger (4) und das Zwischenelement (5) dazu ausgebildet sind, eine Rastverbindung miteinander einzugehen.

9. Analyseeinheit (1) nach einem der Ansprüche 2 bis 8, wobei der Sensorträger (4) im Lagerbereich (2) gegen unbeabsichtigtes Verschieben in den Probenbereich (3) gesichert ist.

10. Analyseeinheit (1) nach einem der vorhergehenden Ansprüche, wobei die Barriere (24) zwischen dem Lagerbereich (2) und dem Probenbereich (3) durch einen Teil einer Wandung des Lagerbereichs (2) gebildet ist.

11. Analyseeinheit (1) nach einem der vorhergehenden Ansprüche, wobei die Barriere (24) zwischen dem Lagerbereich (2) und dem Probenbereich (3) eine Sollbruchstelle aufweist.

12. Analyseeinheit (1) nach einem der vorhergehenden Ansprüche, wobei der Lagerbereich (2) einen Ausweichbereich (23) umfasst, welcher dazu vorgesehen ist, bei Verschiebung des Sensors (6) aus dem Lagerbereich (2) in den Probenbereich (3) verdrängtes Lagermedium (21) aufzunehmen.

13. Analyseeinheit (1) nach einem der vorhergehenden Ansprüche, wobei der Lagerbereich (2) durch eine Folie (22) begrenzt ist.

14. Analyseeinheit (1) nach einem der vorhergehenden Ansprüche, wobei im Lagermedium (21) eine definierte Osmolalität eingestellt ist.

15. Analyseeinheit (1) nach einem der vorhergehenden Ansprüche, wobei das Lagermedium (21) zusätzlich als Kalibriermedium wirkt.

16. Analyseeinheit (1) nach einem der vorhergehenden Ansprüche, wobei der Probenbereich (3) ein Durchflusselement ist.

## Claims

1. Analysis unit (1) having at least one sensor (6),
wherein in a storage state of the analysis unit (1), the at least one sensor (6) is arranged in a storage medium (21) and is wetted through by the storage medium (21);
wherein in a measurement state of the analysis unit (1), the at least one sensor (6) can be brought into contact with a sample;
**characterized in that**
the analysis unit (1) can be moved from the storage state into the measurement state by displacing the at least one sensor (6) from a storage region (2) of the analysis unit (1) containing the storage medium (21) into a sample region (3) of the analysis unit (1), wherein a barrier (24) between the storage region (2) and the sample region (3) is broken through.

2. Analysis unit (1) according to claim 1, wherein the at least one sensor (6) is arranged on a sensor carrier (4), which is designed to break through the barrier (24) between the storage region (2) and the sample region (3).

3. Analysis unit (1) according to claim 2, wherein the at least one sensor (6) is designed to be optically excited and to provide an optical response dependent on an analyte, and the sensor carrier (4) is transparent to the light involved in the optical excitation and the optical response.

4. Analysis unit (1) according to claim 3, wherein a channel (43) for a light guide is provided in the sensor carrier (4).

5. Analysis unit (1) according to one of claims 2 to 4, wherein the storage region (2) is connected to the sample region (3) by an intermediate element (5) in which a receptacle (51) for the sensor carrier (4) is provided to accommodate the sensor carrier (4) in the measurement state.

6. Analysis unit (1) according to claim 5, wherein at least one free space (54) is provided in the receptacle (51) for the sensor carrier (4) for accommodating at least a part of the perforated barrier (24).

7. Analysis unit (1) according to claim 5 or 6, wherein a sealing ring (53) is provided in the intermediate element (5) in order to seal the intermediate element (5) against the sensor carrier (4).

8. Analysis unit (1) according to one of claims 5 to 7, wherein the sensor carrier (4) and the intermediate element (5) are designed to form a snap-in connection with one another.

9. Analysis unit (1) according to one of claims 2 to 8, wherein the sensor carrier (4) is secured in the storage region (2) against unintentional displacement into the sample region (3).

10. Analysis unit (1) according to one of the preceding claims, wherein the barrier (24) between the storage region (2) and the sample region (3) is formed by a part of a wall of the storage region (2).

11. Analysis unit (1) according to one of the preceding claims, wherein the barrier (24) between the storage region (2) and the sample region (3) has a predetermined breaking point.

12. Analysis unit (1) according to one of the preceding claims, wherein the storage region (2) comprises an evasion region (23) which is provided to receive displaced storage medium (21) when the sensor (6) is displaced from the storage region (2) into the sample region (3).

13. Analysis unit (1) according to one of the preceding claims, wherein the storage region (2) is delimited by a foil (22).

14. Analysis unit (1) according to one of the preceding claims, wherein a defined osmolality is set in the storage medium (21).

15. Analysis unit (1) according to one of the preceding claims, wherein the storage medium (21) additionally acts as a calibration medium.

16. Analysis unit (1) according to one of the preceding claims, wherein the sample region (3) is a flow element.

## Revendications

1. Unité d'analyse (1) comportant au moins un capteur (6),
dans laquelle, dans un état de stockage de l'unité d'analyse (1), ledit au moins un capteur (6) est disposé dans un milieu de stockage (21) et est humidifié par le milieu de stockage (21) ;
dans laquelle, dans un état de mesure de l'unité d'analyse (1), ledit au moins un capteur (6) peut être mis en contact avec un échantillon ;
**caractérisée en ce que**
l'unité d'analyse (1) peut être passée de l'état de stockage à l'état de mesure en déplaçant ledit au moins un capteur (6) d'une zone de stockage (2) de l'unité d'analyse (1) contenant le milieu de stockage (21) dans une zone d'échantillon (3) de l'unité d'analyse (1), une barrière (24) entre la zone de stockage (2) et la zone d'échantillon (3) étant rompue.

2. Unité d'analyse (1) selon la revendication 1, dans laquelle ledit au moins un capteur (6) est disposé sur un support de capteur (4) qui est conçu pour rompre la barrière (24) entre la zone de stockage (2) et la zone d'échantillon (3).

3. Unité d'analyse (1) selon la revendication 2, dans laquelle ledit au moins un capteur (6) est conçu pour être excité optiquement et pour fournir une réponse optique dépendant d'un analyte, et le support de capteur (4) est transparent à la lumière impliquée dans l'excitation optique et la réponse optique.

4. Unité d'analyse (1) selon la revendication 3, dans laquelle un canal (43) pour un guide de lumière est prévu dans le support de capteur (4).

5. Unité d'analyse (1) selon l'une des revendications 2 à 4, dans laquelle la zone de stockage (2) est reliée à la zone d'échantillon (3) par un élément intermédiaire (5) dans lequel est prévu un réceptacle (51) pour le support de capteur (4) afin de recevoir le support de capteur (4) dans l'état de mesure.

6. Unité d'analyse (1) selon la revendication 5, dans laquelle au moins un espace libre (54) destiné à recevoir au moins une partie de la barrière rompue (24) est prévu dans le réceptacle (51) pour le support de capteur (4).

7. Unité d'analyse (1) selon la revendication 5 ou 6, dans laquelle une bague d'étanchéité (53) est prévue dans l'élément intermédiaire (5) afin de rendre l'élément intermédiaire (5) étanche par rapport au support de capteur (4).

8. Unité d'analyse (1) selon l'une des revendications 5 à 7, dans laquelle le support de capteur (4) et l'élément intermédiaire (5) sont conçus pour former entre eux une liaison par encliquetage.

9. Unité d'analyse (1) selon l'une des revendications 2 à 8, dans laquelle le support de capteur (4) est bloqué dans la zone de stockage (2) pour éviter un déplacement involontaire dans la zone d'échantillon (3).

10. Unité d'analyse (1) selon l'une des revendications précédentes, dans laquelle la barrière (24) entre la zone de stockage (2) et la zone d'échantillon (3) est formée par une partie d'une paroi de la zone de stockage (2).

11. Unité d'analyse (1) selon l'une des revendications précédentes, dans laquelle la barrière (24) entre la zone de stockage (2) et la zone d'échantillon (3) présente une zone destinée à la rupture.

12. Unité d'analyse (1) selon l'une des revendications précédentes, dans laquelle la zone de stockage (2) comprend une zone de dégagement (23) qui est prévue pour recevoir le milieu de stockage (21) refoulé lors du déplacement du capteur (6) de la zone de stockage (2) vers la zone d'échantillon (3).

13. Unité d'analyse (1) selon l'une des revendications précédentes, dans laquelle la zone de stockage (2) est délimitée par un film (22).

14. Unité d'analyse (1) selon l'une des revendications précédentes, dans laquelle une osmolalité définie est réglée dans le milieu de stockage (21).

15. Unité d'analyse (1) selon l'une des revendications précédentes, dans laquelle le milieu de stockage (21) agit en outre comme milieu de calibrage.

16. Unité d'analyse (1) selon l'une des revendications précédentes, dans laquelle la zone d'échantillon (3) est un élément d'écoulement.
